# EUROPEAN PATENT APPLICATION

(11) **EP 2 087 887 A1**
(43) Date of publication of application: **12.08.2009**
(21) Application number: 07829359.4
(22) Date of filing: 05.10.2007
(51) Int. Cl.: A61K 31/122, A23K 1/16, A23L 1/03, A61K 9/20, A61K 9/48, A61K 47/14, A61K 47/24, A61P 9/04, A61P 17/00

(54) **HIGHLY ABSORBABLE COMPOSITION FOR ORAL ADMINISTRATION CONTAINING OXIDIZED COENZYME Q10**

(30) Priority: 06.10.2006 JP 2006275501; 06.10.2006 US 828490 P
(71) Applicant: Kaneka Corporation, Kita-ku Osaka-shi, Osaka 530-8288 (JP)
(72) Inventor: SHINAGAWA, Yoshiyuki, Akashi-shi, Hyogo 674-0051 (JP); KAWABE, Taizo, Himeji-shi, Hyogo 672-8044 (JP); KISHIDA, Hideyuki, Kakogawa-shi, Hyogo 675-0039 (JP); HOSOE, Kazunori, Takasago-shi, Hyogo 676-0025 (JP)
(74) Representative: Hart-Davis, Jason
(86) International application number: PCT/JP2007/069621
(87) International publication number: WO 2008/044659

(57) **Abstract**

The present invention provides a method of increasing the absorbability of oxidized coenzyme Q₁₀ by preparing the oxidized coenzyme Q₁₀ as a composition in the presence of a lysolecithin and an oil and fat. The composition of the present invention comprising oxidized coenzyme Q₁₀, a lysolecithin and an oil and fat, wherein the weight ratio of the lysolecithin to the oxidized coenzyme Q₁₀ is not less than 0.7, shows superior absorption of oxidized coenzyme Q₁₀ and can be utilized for food, food with nutrient function, food for specified health uses, nutritional supplement, nutritional product, animal drug, beverage, feed, pet food, cosmetics, pharmaceutical product, therapeutic drug, preventive drug and the like.

## Description

### Technical Field

The present invention relates to a composition of high oral absorbability for oral administration comprising oxidized coenzyme Q₁₀, and a method of increasing the absorbability of oxidized coenzyme Q₁₀.

### Background Art

Coenzyme Q, an essential component of the body found in a broad range of organisms, from bacteria to mammals, is known to constitute the mitochondrial electron transfer system in the cells thereof. In the human being, the major component of coenzyme Q is coenzyme Q₁₀, having 10 repeat units in the side chain of coenzyme Q. Coenzyme Q occurs in two types: oxidized and reduced. In the living body, the former normally accounts for about 10 to 60% of the coenzyme Q in the body. Oxidized coenzyme Q has a variety of uses, including drugs for congestive heart failure based on the cardiac effect thereof. Oxidized coenzyme Q₁₀ is also approved for use in general foods. In addition to pharmaceutical uses, oxidized coenzyme Q₁₀ is commercially available in the form of soft capsules, tablets, hard capsules, granules, and beverages. Other uses, like those of vitamins, include nutritional products, nutritional supplements and other oral preparations, as well as dermatological preparations. In particular, many products in the form of soft capsules are available.

Investigations of compositions for oral administration comprising oxidized coenzyme Q have been focusing on the improvement of the temporal stability, temperature stability, and light stability of the composition or preparation, or technology for solubilization to obtain stable and fine emulsion particle diameters.
Meanwhile, oxidized coenzyme Q₁₀ is poorly absorbable as is because it is slightly soluble in water (oil-soluble); no sufficient effect is obtained simply by orally taking oxidized coenzyme Q₁₀ as is. When orally taken in hungry, in particular, oxidized coenzyme Q₁₀ is known to be hardly absorbed.

Against this background, many studies, including investigations of pharmaceutical making for improving the absorbability of oxidized coenzyme Q₁₀, have been conducted to date.
For example, it has been reported that a composition comprising oxidized coenzyme Q₁₀ and a hydrophilic polyhydric alcohol fatty acid ester possesses high absorbability (patent reference 1).

Another such composition disclosed is a composition comprising oxidized coenzyme Q₁₀ at a high content, and further comprising oxidized coenzyme Q₁₀, decaglycerol pentaoleate and diacetylmonocaprin in a particular ratio for the purpose of improving the absorbability (patent reference 2).
Also disclosed is a composition with improved absorbability comprising oxidized coenzyme Q₁₀, an oil phase ingredient, a polyhydric alcohol and an emulsifier (patent reference 3).
Also disclosed is an aqueous solution with improved absorbability prepared by solubilizing an oil-soluble vitamin such as oxidized coenzyme Q₁₀ in the presence of a surfactant to obtain a particle diameter of not more than 110 nm (patent reference 4).

Meanwhile, a composition prepared by finely dispersing an oil and fat comprising oxidized coenzyme Q₁₀ in an emulsion is described as being highly effective in preventing the crystallization and precipitation, with the dislosure of an emulsifier used for this purpose, enzyme-decomposed lecithin (lysolecithin), decaglycerol monolaurate, decaglycerol monooleate and the like (patent reference 5).
patent reference 1: JP-2005-43
patent reference 2: JP-6-65645
patent reference 3: JP-2003-238396
patent reference 4: JP-2004-175664
patent reference 5: JP-2003-300870

### Disclosure of the Invention

### Problems to Be Solved by the Invention

However, the hydrophilic polyhydric alcohol fatty acid esters mentioned as examples in the foregoing patent reference 1 are variable, and the choice of hydrophilic polyhydric alcohol fatty acid esters that significantly contribute to the absorbability, suitable combinations and the like remain unknown. For example, the combinations described in Examples therein are quite complicated, and an actual evaluation by the present inventors revealed that the combinations were not sufficiently effective in obtaining the desired absorbability.

Oxidized coenzyme Q₁₀ tends to crystallize in capsular preparations; it seems difficult to meet all the requirements for stabilizing the capsular preparations, stabilizing oxidized coenzyme Q₁₀, and improving the absorbability, simply by applying the method of improving the absorbability of general oil-soluble substances disclosed in patent reference 1.

Other conventional reports are associated with some problems, for example, no demonstrative data on a significant improvement in the absorbability of oxidized coenzyme Q₁₀ are available so that a reliable formulation for the purpose of oral absorbability is not disclosed, and applicability or practicability is lacked due to the limitation on the choice of oil and fat and surfactant ingredients.

The present invention is intended to provide a composition for oral administration comprising oxidized coenzyme Q₁₀ of high oral absorbability.

### Means of Solving the Problems

The present inventors investigated to solve the above-described problems, and found a composition with improved absorbability of oxidized coenzyme Q₁₀ can be obtained by preparing a composition comprising oxidized coenzyme Q₁₀, a lysolecithin and an oil and fat, wherein the oxidized coenzyme Q₁₀ and the lysolecithin are present in a particular ratio, an outstanding feature of this invention. The present inventors also found that the absorbability of oxidized coenzyme Q₁₀ can be further improved by adding a surfactant.
Accordingly, the present invention relates to the following.
[1] A composition for oral administration comprising an oxidized coenzyme Q₁₀ represented by the structural formula (1):

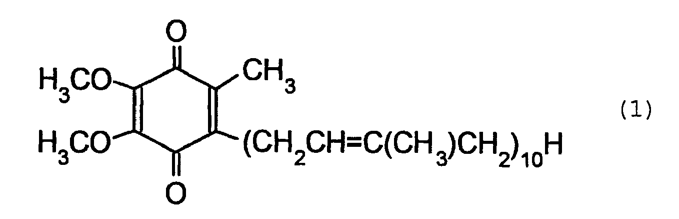

a lysolecithin and an oil and fat, wherein the weight ratio of the lysolecithin to the oxidized coenzyme Q₁₀ is not less than 0.7.
[2] The composition for oral administration of the above-mentioned [1], wherein the weight ratio of the lysolecithin to the oxidized coenzyme Q₁₀ is not less than 1.2.
[3] The composition for oral administration of the above-mentioned [1] or [2], further comprising a lecithin.
[4] The composition for oral administration of the above-mentioned [3], wherein the weight ratio of the total of the lysolecithin and the lecithin to the oxidized coenzyme Q₁₀ is not less than 1.2.
[5] The composition for oral administration of any one of the above-mentioned [1] to [4], wherein the lysolecithin is a lysolecithin derived from at least one kind of lecithin selected from the group consisting of soybean lecithin, egg-yolk lecithin, corn lecithin, cottonseed oil lecithin, rapeseed lecithin, phosphatidylcholine, phosphatidylethanolamine and phosphatidylglycerol.
[6] The composition for oral administration of any one of the above-mentioned [3] to [5], wherein the lecithin is at least one kind of lecithin selected from the group consisting of soybean lecithin, egg-yolk lecithin, corn lecithin, cottonseed oil lecithin, rapeseed lecithin, phosphatidylcholine, phosphatidylethanolamine and phosphatidylglycerol.
[7] The composition for oral administration of any one of the above-mentioned [1] to [6], wherein the oil and fat is at least one kind of oil and fat selected from the group consisting of coconut oil, palm oil, palm kernel oil, linseed oil, camelia oil, unmilled rice germ oil, avocado oil, rapeseed oil, rice oil, peanut oil, corn oil, wheat germ oil, soybean oil, perilla oil, cottonseed oil, sunflower oil, kapok oil, evening primrose oil, shea butter, sal butter, cacao butter, sesame oil, safflower oil, olive oil, almond oil, lard, milk fat, fish oil, beef tallow, processed oils and fats therefrom, middle-chain fatty acid triglycerides, and hydrolysates thereof.
[8] The composition for oral administration of the above-mentioned [7], wherein the oil and fat is at least one kind of oil and fat selected from the group consisting of safflower oil, almond oil and cottonseed oil.
[9] The composition for oral administration of any one of the above-mentioned [1] to [6], wherein the oil and fat is an oil and fat wherein oleic acid accounts for not less than 30 wt% of the constituent fatty acids thereof.
[10] The composition for oral administration of any one of the above-mentioned [1] to [9], further comprising a surfactant.
[11] The composition for oral administration of the above-mentioned [10], wherein the surfactant is at least one kind of surfactant selected from the group consisting of glycerol fatty acid esters, sucrose fatty acid esters, organic acid monoglycerides, sorbitan fatty acid esters, polyoxyethylene sorbitan fatty acid esters, propylene glycol fatty acid esters, condensed ricinoleinic acid polyglycerides, and saponin.
[12] The composition for oral administration of the above-mentioned [11], wherein the surfactant is at least one kind of glycerol fatty acid ester and organic acid monoglyceride.
[13] The composition for oral administration of the above-mentioned [12], wherein the glycerol fatty acid ester is hexaglycerol monooleate and/or tetraglycerol monolaurate.
[14] The composition for oral administration of the above-mentioned [12], wherein the organic acid monoglyceride is monoglyceride citrate.
[15] The composition for oral administration of any one of the above-mentioned [1] to [14], wherein the content of oxidized coenzyme Q₁₀ in the composition is not less than 0.01 wt% to total weight of the composition.
[16] The composition for oral administration of any one of the above-mentioned [1] to [15], which is in the form of a liquid or a slurry.
[17] The composition for oral administration of any one of the above-mentioned [1] to [15], which is in the form of a solid.
[18] The composition for oral administration of any one of the above-mentioned [1] to [17], which is a pharmaceutical or a quasi-drug.
[19] The composition for oral administration of any one of the above-mentioned [1] to [17], which is a food.
[20] The composition for oral administration of any one of the above-mentioned [1] to [17], which is a feed, a prey or a pet food.
[21] The composition for oral administration of any one of the above-mentioned [17] to [20], which is in the form of tablets, powders, chewable tablets, pills, or capsules.
[22] The composition for oral administration of the above-mentioned [21], wherein the capsules are a soft capsule preparation.
[23] A composition for oral administration comprising oxidized coenzyme Q₁₀, a lysolecithin, an oil and fat, and monoglyceride citrate.
[24] A composition for oral administration comprising oxidized coenzyme Q₁₀, a lysolecithin, an oil and fat, and hexaglycerol monooleate.
[25] A composition for oral administration comprising oxidized coenzyme Q₁₀, a lysolecithin, an oil and fat, and tetraglycerol monolaurate.
[26] The composition for oral administration of any one of the above-mentioned [23] to [25], wherein the weight ratio of the lysolecithin to the oxidized coenzyme Q₁₀ is not less than 0.25.
[27] The composition for oral administration of any one of the above-mentioned [23] to [26], wherein the oil and fat is at least one kind of oil and fat selected from the group consisting of safflower oil, almond oil, and cottonseed oil.
[28] The composition for oral administration of any one of the above-mentioned [23] to [26], wherein the oil and fat is an oil and fat wherein oleic acid accounts for not less than 30 wt%of the constituent fatty acids thereof.
[29] The composition for oral administration of any one of the above-mentioned [23] to [28], which is in the form of a soft capsule preparation.
[30] A method of increasing the absorbability of oxidized coenzyme Q₁₀, comprising preparing the oxidized coenzyme Q₁₀ as a composition ingestible in the presence of a lysolecithin in an amount by weight not less than 0.7 times the amount thereof and an oil and fat.
[31] A method of increasing the absorbability of oxidized coenzyme Q₁₀, comprising preparing the oxidized coenzyme Q₁₀ as a composition ingestible in the presence of a lysolecithin, an oil and fat, and monoglyceride citrate.
[32] A method of increasing the absorbability of oxidized coenzyme Q₁₀, comprising preparing the oxidized coenzyme Q₁₀ as a composition ingestible in the presence of a lysolecithin, an oil and fat, and hexaglycerol monooleate.
[33] A method of increasing the absorbability of oxidized coenzyme Q₁₀, comprising preparing the oxidized coenzyme Q₁₀ as a composition ingestible in the presence of a lysolecithin, an oil and fat, and tetraglycerol monolaurate.

### Effect of the Invention

According to the present invention, a composition comprising oxidized coenzyme Q₁₀ of high oral absorbability, which has not been achieved conventionally, can be provided simply by preparing a composition for oral administration comprising oxidized coenzyme Q₁₀, a lysolecithin and an oil and fat, wherein the oxidized coenzyme Q₁₀ and the lysolecithin are present in a particular weight ratio. Conventionally, to improve the absorbability of oxidized coenzyme Q₁₀, it has been necessary to use large amounts of oil and fat and surfactant; the composition of the present invention is useful for consumers in need of calorie intake reduction, because it exhibits higher absorbability at low oil and fat contents, than in the absence of lysolecithin. Also because the high absorbability is assured even during hunger (fasting), the composition of the present invention is useful in that it can be used regardless of the time of ingestion.
By further adding a surfactant, a composition for oral administration having still higher absorbability can be provided.

### Best Mode for Carrying out the Invention

The present invention is hereinafter described in detail with reference to preferred embodiments.
The composition for oral administration of the present invention is a composition for oral administration comprising oxidized coenzyme Q₁₀, a lysolecithin and an oil and fat, wherein the oxidized coenzyme Q₁₀ and the lysolecithin are present in a particular weight ratio (hereinafter also referred to as the composition for oral administration of the present invention), being a composition comprising oxidized coenzyme Q₁₀, and having very high absorbability.

The oxdized coenzyme Q₁₀ used in the present invention can be obtained by, for example, commonly known conventional methods such as synthesis, fermentation, and extraction from natural substances, combined with oxidation reactions as required, and the like. From the viewpoint of safety, ones obtained by fermentation or extraction from natural products are generally preferable.

The lysolecithin used in the present invention may be any one wherein one of the acyl groups of what is called a common lecithin (phospholipid) has been hydrolyzed to a hydroxyl group, and the choice thereof is not subject to limitation. The lysolecithin may also contain non-degraded lecithin. Examples of the lecithin (phospholipid) from which the lysolecithin used in the present invention is derived include natural lecithins such as soybean lecithin, egg-yolk lecithin, corn lecithin, cottonseed oil lecithin, and rapeseed lecithin, as well as phosphatidylcholine, phosphatidylethanolamine, phosphatidylserine, phosphatidylinositol, phosphatidylinositol polyphosphate, phosphatidylglycerol, phosphatidic acid, phosphatidylinositolamine, diphosphatidylglycerol (cardiolipin), or mixtures thereof and the like. In particular, lysolecithins derived from soybean lecithin, egg-yolk lecithin, corn lecithin, cottonseed oil lecithin, rapeseed lecithin, phosphatidylcholine, phosphatidylethanolamine and phosphatidylglycerol are preferable.

Of the above-described lysolecithins, considering the supply stability estimated from the industrial productivity, prices, and product stability, soybean-derived lysolecithin or egg yolk-derived lysolecithin is preferable; considering consumer convenience such as the safety for prevention of food allergies, personal limitations such as religion and creed, and the like, soybean-derived lysolecithin or purified lysophosphatidylcholine, lysophosphatidylethanolamine, lysophosphatidylglycerol, and mixtures thereof are preferable.

In the composition for oral administration of the present invention, the weight ratio of lysolecithin to oxidized coenzyme Q₁₀ must not be less than 0.7, i.e., not less than 0.7 parts by weight of lysolecithin per 1 part by weight of oxidized coenzyme Q₁₀ must be present in the composition. From the viewpoint of increasing the absorbability, the weight ratio of lysolecithin to oxidized coenzyme Q₁₀ is preferably somewhat high; specifically, the ratio is preferably not less than 1, more preferably not less than 1.2, still more preferably not less than 2, particularly preferably not less than 3, most preferably not less than 4. The upper limit is not subject to limitation, and is normally not more than 500; from the viewpoint of pharmaceutical making limitations, the upper limit is preferably not more than 100, more preferably not more than 50; considering the contents of the active ingredient oxidized coenzyme Q₁₀ and other useful co-ingredients and product cost, the upper limit is still more preferably not more than 30, particularly preferably not more than 20, most preferably not more than 10.

The composition for oral administration of the present invention may further comprise a lecithin. In this case, the lecithin content is not subject to limitation, and is preferably not less than 1.2, more preferably not less than 2, still more preferably not less than 3, particularly preferably not less than 4, calculated as the weight ratio of the total of lysolecithin and lecithin to oxidized coenzyme Q₁₀ in the composition. The upper limit is not subject to limitation, and is normally not more than 500; from the viewpoint of pharmaceutical making limitations, the upper limit is preferably not more than 100, more preferably not more than 50; considering the contents of the active ingredient oxidized coenzyme Q₁₀ and other useful co-ingredients, the upper limit is still more preferably not more than 30; considering product cost, the upper limit is particularly preferably not more than 20, most preferably not more than 10.

The lecithin used in the present invention may be any kind of what is called common lecithin (phospholipid). Examples of lecithin (phospholipid) used in the present invention include natural lecithins such as soybean lecithin, egg-yolk lecithin, corn lecithin, cottonseed oil lecithin, and rapeseed lecithin, as well as phosphatidylcholine, phosphatidylethanolamine, phosphatidylserine, phosphatidylinositol, phosphatidylinositol polyphosphates, phosphatidylglycerol, phosphatidic acid, phosphatidylinositolamine, diphosphatidylglycerol (cardiolipin), sphingomyelin, ceramide aminoethylphosphonic acid, ceramide phosphorylglycerol, dicetylphosphoric acid, and stearylamine, or mixtures thereof and the like.

Of the above-described lecithins, soybean lecithin, egg-yolk lecithin, corn lecithin, cottonseed oil lecithin, rapeseed lecithin, phosphatidylcholine, phosphatidylethanolamine and phosphatidylglycerol are preferable; considering supply stability estimated from industrial productivity, prices, and product stability, soybean-derived lecithin or egg yolk-derived lecithin is preferable; considering consumer convenience such as the safety for prevention of food allergies, personal limitations such as religion and creed, and the like, soybean-derived lecithin or purified phosphatidylcholine, phosphatidylethanolamine, phosphatidylglycerol, and mixtures thereof are preferable.

In the composition for oral administration of the present invention, the oxidized coenzyme Q₁₀ content in the composition (oxidized coenzyme Q₁₀ weight/total composition weight) is not subject to limitation, and is normally not less than about 0.01 wt%, preferably not less than about 0.1 wt%, more preferably not less than 1 wt%, particularly preferably not less than 2 wt%, still more preferably not less than 3 wt%, most preferably not less than 5 wt%. Regarding the upper limit, because lysolecithin in an amount exceeding a given level, and an oil and fat, must be contained, the upper limit is not more than about 59 wt%, preferably not more than 50 wt%, more preferably not more than 30 wt%, still more preferably not more than 20 wt%.

The total of the contents of oxidized coenzyme Q₁₀, lysolecithin and lecithin (used as required) in the composition for oral administration of the present invention ((oxidized coenzyme Q₁₀ weight + lysolecithin weight + lecithin weight)/total composition weight) is not subject to limitation, and is, for example, not less than 0.017 wt%, preferably not less than 0.1 wt%, more preferably not less than 1 wt%, still more preferably not less than about 2 wt%, particularly preferably not less than about 3 wt%, most preferably not less than about 5% weight. For the purpose of adding an oil and fat, other active ingredients, and stability- or absorbability-improving ingredients, the upper limit is preferably not more than 90 wt%, more preferably not more than 80 wt%, still more preferably not more than 70 wt%.

The oil and fat used in the present invention may be a natural oil and fat of animal or vegetable origin, and may be a synthetic oil and fat or a processed oil and fat. Preferably, the oil and fat is one acceptable for food use or pharmaceutical use. As examples of the vegetable oil and fat, coconut oil, palm oil, palm kernel oil, linseed oil, camellia oil, unmilled rice germ oil, rapeseed oil, rice oil, peanut oil, almond oil, corn oil, wheat germ oil, soybean oil, perila oil, cottonseed oil, sunflower oil (sunflower seed oil), kapok oil, evening primrose oil, shea butter, sal butter, cacao butter, sesame oil, safflower oil, olive oil, avocado oil, poppy oil, burdock seed oil, almond oil and the like can be mentioned; as examples of the animal oil and fat, lard, milk fat, fish oil, beef tallow and the like can be mentioned; furthermore, oils and fats prepared by processing them by separation, hydrogenation, ester exchange and the like (for example, hydrogenated oil) can also be mentioned. Needless to say, a medium-chain triglyceride (MCT) can also be used. The medium-chain triglyceride is not subject to limitation; for example, a triglyceride wherein each fatty acid has 6 to 12, preferably 8 to 12 carbon atoms, and the like can be mentioned. A hydrolysate thereof (diglyderide or monoglyceride) can also be used. Furthermore, a mixture of these above-mentioned oils and fats may be used.

Of the above-described oils and fats, coconut oil, palm oil, palm kernel oil, linseed oil, camelia oil, unmilled rice germ oil, avocado oil, rapeseed oil, rice oil, peanut oil, corn oil, wheat germ oil, soybean oil, perilla oil, cottonseed oil, sunflower oil, kapok oil, evening primrose oil, shea butter, sal butter, cacao butter, sesame oil, safflower oil, olive oil, almond oil, lard, milk fat, fish oil, beef tallow, and processed oils and fats therefrom, middle-chain fatty acid triglycerides, and fatty acid partial glycerides are preferable; from the viewpoint of ease of handling, odor and the like, coconut oil, palm oil, palm kernel oil, linseed oil, camelia oil, unmilled rice germ oil, avocado oil, rapeseed oil, rice oil, peanut oil, corn oil, wheat germ oil, soybean oil, perilla oil, cottonseed oil, sunflower oil, kapok oil, evening primrose oil, shea butter, sal butter, cacao butter, sesame oil, safflower oil, almond oil, olive oil, and processed oils and fats therefrom, and middle-chain fatty acid triglycerides are more preferable. Of these oils and fats, considering oil and fat prices, oxidized coenzyme Q₁₀ stability, solubility and the like, coconut oil, palm oil, palm kernel oil, rapeseed oil, rice oil, soybean oil, cottonseed oil, safflower oil, almond oil, olive oil, MCT and the like are preferable, and rice oil, soybean oil, rapeseed oil, cottonseed oil, safflower oil, almond oil, olive oil, MCT and the like are particularly preferable.

The oil and fat in the composition for oral administration of the present invention, the oleic acid content is preferably as high as possible, based on fatty acid residues that constitute the oil and fat contained therein. For example, the oleic acid content in the constituent fatty acids is preferably not less than 30 wt%, more preferably not less than 40 wt%, still more preferably not less than 50 wt%, particularly preferably not less than 70 wt%. The upper limit is not subject to limitation, and is up to 100 wt%. Such an oil and fat may be chosen from among the oils and fats exemplified above, meeting these conditions; two or more kinds of oil and fat may be blended as appropriate to meet these conditions. Also, an oil and fat exemplified above, as the starting material, may be subjected to a process to increase the oleic acid content of constituent fatty acid composition (e.g., fractionation, ester exchange), and an oil and fat having a high oleic acid content as a constituent fatty acid may be chemically synthesized. For example, safflower oil, which is a natural oil and fat having a high oleic acid content in the constituent fatty acid composition, fractionated oils therefrom, and processed oils and fats therefrom can be used. As such an oil and fat, oils and fats containing high oleic acid, such as safflower oil containing high oleic acid and rapeseed oil containing high oleic acid, can be mentioned; in particular, safflower oil containing high oleic acid is more preferably used. An oil and fat wherein the oleic acid content accounts for not less than about 50% of the constituent fatty acids thereof is called an oil and fat containing high oleic acid.

The oil and fat content in the composition for oral administration of the present invention is not subject to limitation, and is normally not less than 1 wt%, preferably not less than 5 wt%, more preferably not less than 10 wt%, particularly preferably not less than 20 wt%, still more preferably not less than 40 wt%, most preferably not less than 60 wt%. The upper limit is not more than 99 wt%, preferably not more than 98 wt%, more preferably not more than 97 wt%, still more preferably not more than 95 wt%.

When the composition for oral administration of the present invention is prepared as a soft capsule preparation, the weight ratio of the oxidized coenzyme Q₁₀/lysolecithin/ oil and fat in the composition is not subject to limitation, but from the viewpoint of containment of the required amount of oxidized coenzyme Q₁₀, absorbability improving effect, and preparation stability, the ratio is preferably between 1/0.7/8.3 - 1/3/6.

For capsular preparations, in particular, it is preferable to use a stabilizer for the purpose of improving preparation stability and oxidized coenzyme Q₁₀ stability. It is also preferable to contain one kind or more of a surfactant, ethanol, and water as other ingredients. In particular, it is preferable to further contain a surfactant for the purpose of improving the absorbability, reducing the absorbability variation due to individual differences, preventing the precipitation of oxidized coenzyme Q₁₀, and improving stability in the oxidized coenzyme Q₁₀ preparation.

As preferable examples of the surfactant used in combination in the composition for oral administration of the present invention, a glycerol fatty acid ester, a sucrose fatty acid ester, an organic acid monoglyceride, sorbitan fatty acid ester, a polyoxyethylene sorbitan fatty acid ester, a propylene glycol fatty acid ester, a condensed ricinoleic acid polyglyceride, saponin and the like can be mentioned. Of these, a glycerol fatty acid ester, an organic acid monoglyceride and the like are preferable.

The glycerol fatty acid ester is not subject to limitation; any of a monoglycerol fatty acid ester and a polyglycerol fatty acid ester can be used. For example, a glycerol fatty acid ester wherein the degree of polymerization of glycerol is 1 to 10, and each fatty acid residue has 6 to 18 carbon atoms, and the like can be mentioned. The fatty acid residue in the glycerol fatty acid ester is not subject to limitation, whether saturated or unsaturated. The upper limit of the number of fatty acid residues is the number of hydroxyl groups present in the glycerol skeleton (that is, degree of polymerization of glycerol + 2). As examples of such a fatty acid residue, caprylic acid, capric acid, lauric acid, myristic acid, palmitic acid, stearic acid, isostearic acid, oleic acid, linoleic acid, linolenic acid and the like can be mentioned. If two or more fatty acid residues are present, the individual fatty acid residues may be the same or different, but from the viewpoint of the ease of obtainment and the like, they are preferably the same.

The glycerol fatty acid ester is preferably hexaglycerol monooleate, tetraglycerol monolaurate, monoglycerol linoleate, or monoglycerol caprylate, more preferably hexaglycerol monooleate or tetraglycerol monolaurate. These esters may be used singly or in combination.

Sucrose fatty acid ester is not particularly limited, and as the fatty acid residue of sucrose fatty acid ester, any can be used whether saturated or unsaturated. The fatty acid residue preferably has 8 to 22 carbon atoms, particularly preferably 8 to 18 carbon atoms. As such fatty acid residue, for example, caprylic acid, capric acid, lauric acid, myristic acid, palmitic acid, stearic acid, isostearic acid, oleic acid, linoleic acid, linolenic acid, behenic acid and the like can be mentioned. When two or more fatty acid residues are present, they may be the same or different. In view of easy availability and the like, they are preferably the same.

While organic acid monoglyceride is not particularly limited, for example, monoglyceride acetate, monoglyceride citrate, monoglyceride lactate, monoglyceride succinate, monoglyceride tartarate such as monoglyceride diacetyl tartrate and the like, and the like can be mentioned. Of these, monoglyceride citrate is preferable. Here, fatty acid residue constituting organic acid monoglyceride is not particularly limited. For example, caprylic acid, capric acid, lauric acid, myristic acid, palmitic acid, stearic acid, oleic acid, linoleic acid, linolenic acid, behenic acid and the like can be mentioned. Of these, preferred are myristic acid, palmitic acid, stearic acid, oleic acid and the like.

Sorbitan fatty acid ester is not particularly limited, and as the fatty acid residue of sorbitan fatty acid ester, any can be used whether saturated or unsaturated. The fatty acid residue preferably has 8 to 22 carbon atoms, particularly preferably 8 to 18 carbon atoms. As such fatty acid residue, for example, caprylic acid, capric acid, lauric acid, myristic acid, palmitic acid, stearic acid, isostearic acid, oleic acid, linoleic acid, linolenic acid, behenic acid and the like can be mentioned, with particularly preference given to oleic acid. When two or more fatty acid residues are present, they may be the same or different. In view of easy availability and the like, they are preferably the same.

Polyoxyethylene sorbitan fatty acid ester is not particularly limited, and as the fatty acid residue of polyoxyethylene sorbitan fatty acid ester, any can be used whether saturated or unsaturated. The fatty acid residue preferably has 8 to 22 carbon atoms, particularly preferably 8 to 18 carbon atoms. As such fatty acid residue, for example, caprylic acid, capric acid, lauric acid, myristic acid, palmitic acid, stearic acid, isostearic acid, oleic acid, linoleic acid, linolenic acid, behenic acid and the like can be mentioned, with particularly preference given to oleic acid. When two or more fatty acid residues are present, they may be the same or different. In view of easy availability and the like, they are preferably the same.

Propylene glycol fatty acid ester is not particularly limited, and any of propylene glycol fatty acid monoester and propylene glycol fatty acid diester can be preferably used. Any fatty acid residue of propylene glycol fatty acid ester can be used whether saturated or unsaturated. For example, the fatty acid residue has 6 to 18 carbon atoms, preferably 6 to 12 carbon atoms. As such propylene glycol fatty acid ester, for example, propylene glycol monocaprylate, propylene glycol dicaprylate, propylene glycol monocaprate, propylene glycol dicaprate, propylene glycol monolaurate, propylene glycol dilaurate, propylene glycol monomyristate, propylene glycol dimyristate, propylene glycol monopalmitate, propylene glycol dipalmitate, propylene glycol monostearate, propylene glycol distearate, propylene glycol monoisostearate, propylene glycol diisostearate, propylene glycol monooleate, propylene glycol dioleate, propylene glycol monolinoleate, propylene glycol dilinoleate, propylene glycol monolinoleate, propylene glycol dilinoleate and the like can be mentioned.

While condensed ricinolein acid polyglyceride is not particularly limited and any can be used irrespective of the degree of polymerization of glycerol and the like. For example, one having a degree of polymerization of 2 to 10 can be mentioned. Preferably, degree of polymerization of glycerol is not less than 2, more preferably not less than 3. While the upper limit of the degree of polymerization of glycerol is not particularly limited, it is normally not more than 10, preferably not more than 8, more preferably not more than 6. As such condensed ricinolein acid polyglycerides, for example, condensed ricinolein acid diglyceride, condensed ricinolein acid triglyceride, condensed ricinolein acid tetraglyceride, condensed ricinolein acid pentaglycerides, condensed ricinolein acid hexaglyceride, condensed ricinolein acid octaglyceride and the like can be mentioned. Preferably, condensed ricinolein acid tetraglyceride, condensed ricinolein acid hexaglyceride and the like can be mentioned.

Examples of the saponin include, but are not limited to, pagoda tree saponin, soapbark saponin, purified soybean saponin, yucca saponin and the like.

The surfactant content in the composition for oral administration of the present invention is not subject to limitation, and is normally not more than 90 wt%, preferably not more than 80 wt%, more preferably not more than 70 wt%, still more preferably not more than 60 wt%. The lower limit is of course 0 wt%; when a surfactant is used, the lower limit is normally not less than 1 wt%, preferably not less than 3 wt%, more preferably not less than 5 wt%, particularly preferably not less than 10 wt%.
When the composition for oral administration of the present invention is prepared as a soft capsule preparation, in particular, the surfactant content in the composition is preferably, but is not limited to, 10 to 30 wt%in the composition.

In another embodiment of the present invention, the composition for oral administration of the present invention can be a composition for oral administration comprising oxidized coenzyme Q₁₀, lysolecithin, an oil and fat, and monoglyceride citrate, a composition for oral administration comprising oxidized coenzyme Q₁₀, lysolecithin, an oil and fat, and hexaglycerol monooleate, or a composition for oral administration comprising oxidized coenzyme Q₁₀, lysolecithin, an oil and fat, and tetraglycerol monolaurate. By preparing a composition comprising the four components of oxidized coenzyme Q₁₀, lysolecithin, an oil and fat, and the above-described particular surfactant, a composition for oral administration having particularly high absorbability of oxidized coenzyme Q₁₀ can be obtained; in this case, the content ratio of oxidized coenzyme Q₁₀ and lysolecithin(lysolecithin/ oxidized coenzyme Q₁₀)is not subject to limitation, and is normally not less than 0.25, preferably not less than 0.7, more preferably not less than 1, still more preferably not less than 1.2, still yet more preferably not less than 2, particularly preferably not less than 3, most preferably not less than 4. The choice of lysolecithin and oil and fat, preferable examples thereof, preferable contents of various ingredients and the like are the same as those described above.

The composition for oral administration of the present invention may further comprise a hydrophilic high-molecular polymer for the purpose of increasing and maintaining the absorbability thereof. The hydrophilic high-molecular polymer may coexist with oxidized coenzyme Q₁₀ in the composition, as long as it is in a form ingested simultaneously with oxidized coenzyme Q₁₀, and may be present as part of, for example, gelatin capsules and preparation ingredients such as coating agents.

Examples of the hydrophilic high-molecular polymer include natural products such as chitin, chitosan, gelatin, casein, β-glucan, trehalose, agar, arginic acid and derivative salts thereof, mannan, pullulan, hyaluronic acid and derivative salts thereof, chondroitin sulfate, dextrin, pectin, starch and derivatives thereof, gum arabic, tragacanth gum, xanthan gum, guar gum, locust bean gum, quince seed, carrageenan, galactan, tara gum, tamarind, furcellaran, karaya gum, sunset hibiscus, cara gum, xanthan gum, gellan gum, and cellulose derivatives such as hydroxypropylcellulose, hydroxypropylmethylcellulose, hydroxyethylcellulose, carboxymethylcellulose, and methylcellulose, and microcrystalline cellulose; semi-synthetic products; and synthetic polymers such as polyvinyl alcohol, polyvinylpyrrolidone, polyvinyl methyl ether, carboxyvinyl polymer, polyacrylates, polymethacrylates, acrylate-polymethacrylates, and polyethylene glycol. Of these substances, it is preferable, from the viewpoint of absorbability improvement, safety, versatility, and ease of preparation making, to use a natural or semi-synthetic hydrophilic high-molecular polymer.

The composition for oral administration of the present invention may be microcapsulated or microsphered (beaded) through the use of these highly hydrophilic polymers to improve the light stability and oxidation stability thereof, to suppress the crystallization thereof, to confer sustained-release quality, enteric quality, and fluidity, to mask the odor and taste thereof, and to resolve the problem of incompatibility; therefore, this embodiment is preferable. Beads are particularly preferable as an embodiment of powdered preparation for the composition for oral administration of the present invention because they are tougher than double-layered microcapsules and hence free from adhesion upon during tableting, and also because they have good fluidity and hence enable easy filling in capsules; they offer good handlability during manufacture and excellent tableting quality.

As long as the effect of the present invention is not affected, another useful compound or extract (bioactive substance or pharmacologically active substance) (hereinafter also referred to as a useful compound and the like) may be contained to obtain a more useful composition for oral administration. For example, a combination of oxidized coenzyme Q₁₀ with a useful compound and the like is considered to exhibit much better utility than with oxidized coenzyme Q₁₀ used alone because of the additive and synergistic effects of combining the useful action mechanisms.

Examples of such other useful ingredients include vitamin A, vitamin B and derivatives thereof, vitamin C and derivatives thereof, vitamin D, vitamin E and derivatives thereof, vitamin K, carotenoids, unsaturated fatty acids, polyphenols, health food materials, nutritional supplementary food materials, glutathione, pyrroloquinolinequinone and pyrroloquinolinequinone derivatives, superoxide dismutase (SOD), glutathione peroxidase, glutathione-S-transferase, glutathione reductase, catalases, ascorbic acid peroxidase, natto kinase, melatonin, curcumin, astaxanthin, N-acetylcysteine and derivatives thereof, taurine, phosphatidylserine, St.-John's-wort, terpenes, garlic extract, toki-syakuyakusan, L-carnitine and derivatives thereof, α-lipoic acid and derivatives thereof, gingko leaf extract, iron preparations, zinc, magnesium, selenium, sodium thiosulfate and the like.

Examples of the vitamin B include thiamin, riboflavin, niacin, pantothenic acid, pyridoxine, cyanocobalamine, folic acid, inositol, para-aminobenzoic acid, biotin and the like.
Examples of the carotenoids include licopene, β-carotene, lutein, zeaxanthin and the like.
Examples of the unsaturated fatty acids include DHA, EPA, arachidonic acid and derivatives thereof, γ-linolenic acid and derivatives thereof and the like.
Examples of the polyphenols include flavonol, isoflavone, tannin, catechin, quercetin, anthocyanin, pycnogenol (flavangenol), flavonoids and the like.

Examples of the health food materials include, but are not limited to kanpo medicines (e.g., ireito, unkeito, unsei'in, ogi-kentyuto, oren-gedokuto, orento, kakkonto, kami-kihito, kami-syoyosan, kanbaku-daisoto, kikyoto, kihito, kyumi-binroto, keigai-rengyoto, keisi-ka-syakuyaku-daioto, keihi-ka-syakuyakuto, keihi-ka-ryukotu-boreito, keisito, keisi-ninzinto, keisibukuryogan, keihito, kososan, gokoto, gosyakusan, gosha-jinkigan, gorinsan, saikanto, saiko-ka-ryukotu-boreito, saiko-keisikankyoto, saiko-keisito, saiko-seikanto, saibokuto, saireito, sansoninto, ziin-kokato, sigyakusan, sikunsito, simotuto, syakanzoto, syakuyakukanzoto, zyuzen-taihoto, zyumi-haidokuto, syoken-tyuto, syosaikoto, syoseiryuto, syohusan, sin'i-seihaito, sinpito, sinbuto, seizyo-bohuto, seisyo-ekkito, seisin-rensiin, seihaito, sokei-kakketuto, daio-kanzoto, daio-botanpito, daikentyuto, daisaikoto, daisaikoto-kyo-daio, daijyokito, daibohuto, jidaboku-ippo, tyoi-zyokito, tyotosan, tyoyoto, tyoreito, tyoreito-go-simotuto, tudosan, tokaku-zyokito, tokiinsi, toki-kentyuto, tokito, nitinto, nyosinsan, ninzinto, ninzin-yoeito, hainosankyuto, bakumondoto, hatimi-ziogan, hange-kobokuto, hange-syasinto, byakko-ka-ninzinto, bukuryoin, bukuryoin-go-hange-kobokuto, heiisan, boi-ogito, bohu-tusyosan, hotyu-ekkito, maoto, mao-busi-saisinto, makyo-kansekito, masiningan, mokuboito, yokukansan, yokukansan-ka-tinpi-hange, rikkunsito, rikkosan, ryutan-syakanto, ryokankyo-mi-singeninto, rokumi-ziogan and the like), tea leaves (e.g., green tea, unmilled rice tea, powdered tea, green tea of middle grade, toasted tea, roasted tea, jasmine tea, oolong tea, hongcha, heicha, huacha, jincha, baicha and the like), herbs (e.g., Italian parsley, elecampane, olive, oregano, cardoon, chamomile, curry plant, catnip, caraway, Christmas rose, crimson clover, cornflower, common mallow, salad burnet, santolina, cinnamon, jasmine, stevia, sage, European linden, scented geranium, soapwort, Solomon's-seal, thyme, tansy, chervil, chive, nasturtium, jujube, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, lady's bedstraw, lemon grass, lemon verbena, lemon balm, rose, rosemary, rocket, wild strawberry, wild pansy, forget-me-not and the like), propolis, aojiru (green-leaved-vegetable juice) and extract thereof and the like.

Examples of the nutritional supplementary food materials include, but are not limited to, amino acids, metal ions, proteins, saccharides, fatty acids, yeast extract, vegetable extract, fish meat extract, fruit, fruit extract and the like.

Examples of the administration (ingestion) form of oxidized coenzyme Q₁₀ and the above-described useful compound and the like preferably used in combination therewith include, but are not limited to, (1) administration of a composition comprising oxidized coenzyme Q₁₀ and a useful compound and the like, i.e., as a single preparation, (2) concurrent administration of two kinds of preparation obtained by separately preparing oxidized coenzyme Q₁₀ and a useful compound and the like via the same route of administration, (3) administration of two kinds of preparation obtained by separately preparing oxidized coenzyme Q₁₀ and a useful compound and the like via the same route of administration at a time lag (e.g., administration in the order of oxidized coenzyme Q₁₀, a useful compound and the like, or administration in the reverse order), (4) concurrent administration of two kinds of preparation obtained by separately preparing oxidized coenzyme Q₁₀ and a useful compound and the like via different routes of administration, (5) administration of two kinds of preparation obtained by separately preparing oxidized coenzyme Q₁₀ and a useful compound and the like via different routes of administration at a time lag (e.g., administration in the order of oxidized coenzyme Q₁₀, a useful compound and the like, or administration in the reverse order) and the like. For example, the administration (ingestion) form includes the use of the composition for oral administration of the present invention for a patient undergoing a treatment using the above-described useful compound and the like.

The composition for oral administration of the present invention may be further blended with pharmaceutically and food-hygienically acceptable other materials as appropriate by a conventional method. Examples of such materials include, but are not limited to, an excipient, disintegrant, lubricant, binder, coating agent, colorant, anticoagulantt, absorption promoter, solubilizing agent, stabilizer, flavor, sweetener, antiseptic, preservative, antioxidant and the like.

Examples of the excipient include, but are not limited to, white soft sugar, lactose, glucose, cornstarch, mannitol, crystalline cellulose, calcium phosphate, calcium sulfate and the like.

Examples of the disintegrant include, but are not limited to, starch, agar, calcium citrate, calcium carbonate, sodium hydrogen carbonate, dextrin, crystalline cellulose, carboxymethylcellulose, tragacanth and the like.

Examples of the lubricant include, but are not limited to, talc, magnesium stearate, polyethylene glycol, silica, hydrogenated oil and the like.

The coating agent may be any one in common use for oral administration preparations; for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, methacrylate-acrylate copolymer (e.g., Eudragit (trade name), Roehm Pharma GmbH, and the like), lactose, gelatin, polyvinyl alcohol, polyvinylacetal diethylamino acetate, shellac and the like can be mentioned. To prepare the preparation for oral administration of the present invention as a rapid-release preparation or sustained-release preparation, a water-soluble coating agent, a gastric coating agent or an enteric coating agent can be chosen, and a commonly known coating agent can also be used for the purpose of taste masking and release sustainability. When an enteric coating agent is used, a commonly known coating agent may be used in combination to provide an intermediate phase between the enteric phase and the drug-containing phase.

Examples of the binder include, but are not limited to, ethylcellulose, methylcellulose, hydroxypropylmethylcellulose, hydroxypropylcellulose, tragacanth, shellac, gelatin, pullulan, gum arabic, polyvinylpyrrolidone, polyvinyl alcohol, polyacrylic acid, polymethacrylic acid, sorbitol and the like.

Examples of the colorant include, but are not limited to, those approved for use as additives in pharmaceuticals or foods and the like can be used; examples include pigments such as Blue No. 1, Yellow No. 4, Green No. 3, Red No. 5, lake pigments, titanium dioxide, red cabbage pigment, beni-koji pigment, purple sweet potato pigment, gardenia pigment, cochineal pigment, red iron oxide pigment, safflower pigment, caramel pigment, tar pigment, and chlorophyll, and the like.

Examples of the anticoagulantt include, but are not limited to, stearic acid, talc, light silicic anhydride, hydrated silicon dioxide and the like.

Examples of the absorption promoter include, but are not limited to, higher alcohols, higher fatty acids and the like.

Examples of the solubilizing agent include, but are not limited to, fumaric acid, succinic acid, malic acid, adipic acid, L-arginine, sodium benzoate, benzyl benzoate, esterified corn oil, ethanol, magnesium chloride, hydrochloric acid, olive oil, carmellose sodium, dry sodium carbonate, dilute hydrochloric acid, citric acid, sodium citrate, glycine, glycerol, geraniol, acetic/phthalic acid cellulose, sodium salicylate, magnesium oxide, α-cyclodextrin, β-cyclodextrin, γ-cyclodextrin, dibutylhydroxytoluene, tartaric acid, sodium hydroxide, sorbitan sesquioleate, D-sorbitol, D-sorbitol solutions, sodium hydrogen carbonate, sodium carbonate, triacetin, sorbitan trioleate, nicotinic amide, lactic acid, concentrate glycerol, copper-nickel alloy, hydroxypropylmethylcellulose, castor oil, glacial acetic acid, glucose, propylene glycol, povidone, polyoxyethylene hydrogenated castor oil, polyoxyethylene (160) polyoxypropylene (30) glycol, polysorbate, polyvinyl alcohol, macrogol, D-mannitol, isopropyl myristate, sorbitan monooleate, lauryl macrogol, lidocaine, phosphoric acid, sodium hydrogen phosphate, potassium dihydrogen phosphate and the like.

Examples of the stabilizer include, but are not limited to, benzoic acid, sodium benzoate, ethyl para-oxybenzoate, yellow beeswax, hydroxypropylmethylcellulose, and methylcellulose.

Examples of the flavor include single-ingredient flavors such as menthol, carvone, anethole, cineole, methyl salicylate, cinnamic aldehyde, eugenol, 3,1-menthoxypropane-1,2-diol, thymol, linalol, linalyl acetate, limonene, menthone, menthyl acetate, N-subsituted-para-menthane-3-carboxamide, pinene, octylaldehyde, citral, pulegone, carvyl acetate, anise aldehyde, ethyl acetate, ethyl butyrate, arylcyclohexane propionate, methyl anthranylate, ethylmethylethinyl glycidate, vanillin, undecalactone, hexanal, ethyl alcohol, propyl alcohol, butanol, isoamyl alcohol, hexenol, dimethyl sulfide, cyclotene, furfural, trimethylpyrazone, ethyl lactate, and ethyl thioacetate; as well as natural flavors such as peppermint oil, spearmint oil, anise oil, eucalyptus oil, wintergreen oil, cassia oil, clove oil, thyme oil, sage oil, lemon oil, orange oil, mentha oil, cardamom oil, coriander oil, mandarin oil, lime oil, lavender oil, rosemary oil, laurel oil, chamomile oil, caraway oil, marjoram oil, bay oil, lemon-grass oil, origanum oil, pine needle oil, neroli oil, rose oil, jasmine oil, iris concrete, absolute peppermint, absolute rose, and orange flower; blended flavors such as strawberry flavor, apple flavor, banana flavor, pineapple flavor, grape flavor, mango flavor, butter flavor, milk flavor, fruit mix flavor, and tropical fruit flavor, and the like.

Examples of the sweetener include saccharin sodium, aspartame, stevioside, stevia extract, para-methoxycinnamic aldehyde, neohesperidyl dihydrochalcone, perilla rutin and the like.

Examples of the antiseptic include aminoethylsulfonic acid, benzoic acid, sodium benzoate, ethanol, sodium edetate, agar, dl-camphor, citric acid, sodium citrate, salicylic acid, sodium salicylate, phenyl salicylate, dibutylhydroxytoluene, sorbic acid, potassium sorbate, nitrogen, dehydroacetic acid, sodium dehydroacetate, 2-naphthol, white soft sugar, honey, isobutyl para-oxybenzoate, isopropyl para-oxybenzoate, ethyl para-oxybenzoate, butyl para-oxybenzoate, propyl para-oxybenzoate, methyl para-oxybenzoate, 1-menthol, eucalyptus oil and the like.

Examples of the preservative include benzoic acid, sodium benzoate, ethanol, sodium edetate, dry sodium sulfite, citric acid, glycerol, salicylic acid, sodium salicylate, dibutylhydroxytoluene, D-sorbitol, sorbic acid, potassium sorbate, sodium dehydroacetate, isobutyl para-oxybenzoate, isopropyl para-oxybenzoate, ethyl para-oxybenzoate, butyl para-oxybenzoate, propyl para-oxybenzoate, methyl para-oxybenzoate, propylene glycol, phosphoric acid and the like.

Examples of the antioxidant include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α lipoic acid and derivatives thereof, pycnogenol, flavangenol, superoxide dismutase (SOD), glutathione peroxidase, glutathione-S-transferase, glutathione reductase, catalases, ascorbic acid peroxidase, and mixtures thereof and the like. In particular, antioxidants are used as additives particularly preferably from the viewpoint of increasing the oxidation stability of the ingredients contained in the composition, and hence obtaining stabler preparations.

The composition for oral administration of the present invention may be in the state of a solid (powder, granule and the like) or a liquid (solution). Here, the liquid state may be a melt of oxidized coenzyme Q₁₀, or a state where other liquid component is present, and oxidized coenzyme Q₁₀ and/or lysolecithin is dissolved in the liquid component. Needless to say, it may be a slurry wherein a part of oxidized coenzyme Q₁₀ and/or lysolecithin is precipitated.
The method of powdering/granulating the composition for oral administration of the present invention is not subject to limitation; methods in common use, such as spray drying, kneading granulation, and freeze-drying, can be used, and the powder and granules thus prepared can be prepared as a variety of preparations such as granules, hard capsules, and chewable tablets, as described below.

The dosage, administration, delivery technology, carrier for delivery or formulation and the like for the composition of the present invention are not subject to limitation, as long as the composition is orally administered; considering the efficacy, convenience, economics, versatility, safety and the like, these can be determined as appropriate, as long as the effect of the invention is not affected. Depending on the compound or extract to be used in combination or in mixture, or depending on the patient or consumer to receive or ingest the composition of the present invention, the dosage, administration, delivery technology, carrier for delivery or formulation and the like can be changed as appropriate, as long as the effect of the invention is not affected, considering the efficacy, convenience, economics, versatility, safety and the like. Examples of the subject of administration or ingestion include mammals, for example, primates, including humans, horses, birds, bovines, pigs, dogs, cats, and murines, with preference given to humans.

The composition for oral administration of the present invention can be used as a pharmaceutical or a quasi-drug as is, or with other pharmaceutically acceptable ingredients. When the composition for oral administration of the present invention is used as a pharmaceutical or a quasi-drug, applicable dosage forms include various preparations for oral administration, specifically tablets, pills, capsules, powders, chewable tablets and the like. Preferably, the composition for oral administration of the present invention is prepared as capsules, more preferably as a soft capsule preparation.

The composition for oral administration of the present invention can also be used as a food as is, or with other foods and/or acceptable ingredients. To facilitate ingestion, the composition for oral administration of the present invention may be prepared as processed foods with the addition of ordinary food materials and additives such as seasonings and flavorings. Here, the foods in the present invention include general foods, including health foods, as well as functional foods, including foods for specified health uses or foods with nutrient function claims.

The choice of the food is not subject to limitation; from the viewpoint of the routine, effective, and long-term ingestion of oxidized coenzyme Q₁₀, the composition for oral administration of the present invention is preferably used in liquid foods such as beverages (e.g., refreshing beverages, milk beverages, vegetable/fruit juice beverages, spirits, tea), drinkable preparations, and soups; milky or pasty foods such as curry; semi-solid foods such as jellies and "gumi" candies; spontaneously liquid diets, semi-digested nutritional foods and component nutrient foods, nutritional products, nutritional supplements, various supplements for oral administration (tablets, pills, capsules, powders, chewable tablets) and the like. In the case of health foods, a form wherein oxidized coenzyme Q₁₀ is packed in a unit ingestion volume per serving and the like can be mentioned; in the case of health drinks, a drink wherein oxidized coenzyme Q₁₀ is suspended or dissolved is supplied for a single-consumption unit in a bottle or the like can be mentioned.

The composition for oral administration of the present invention is widely used in pharmaceuticals, a quasi-drug, foods, animal pharmaceuticals, animal supplements, feeds, pet foods, and the like.

When used in the above-described pharmaceuticals, foods and the like, the composition for oral administration of the present invention can be used in the form of aqueous solution, powder, liquid and the like chosen as appropriate according to the shape and intended use thereof.

The composition for oral administration of the present invention can be used for a variety of purposes for improving the quality of life (QOL) of humans, including the prevention and treatment for various diseases, reduction of side reactions, promotion of recovery from disease and the like, and can also be used for the purpose of maintaining and promoting daily health and the like. The dosage of the composition for oral administration of the present invention is not subject to limitation, and is preferably 1 to 1200 mg per day for a human, based on the amount of oxidized coenzyme Q₁₀, and from the viewpoint of utility and economics, it is more preferably 10 to 800 mg, and from the viewpoint of routine ingestion and onset of effects, it is particularly preferably 30 to 300 mg. The above-described daily amount can be taken at one time or in several divided portions. Duration of ingestion is not subject to limitation.

Because the composition for oral administration of the present invention has high absorbability, the various useful effects of oxidized coenzyme Q₁₀ can be exhibited in full, so that it is effective as beverages/foods, pharmaceuticals, and a quasi-drug and the like. The composition for oral administration of the present invention is also effective as beverages/foods, health foods, functional foods, food for specified health uses, foods for babies and infants, foods for the aged and the like for the purpose of improving daily QOL.

The present invention also provides a method of increasing the absorbability of oxidized coenzyme Q₁₀ by ingesting oxidized coenzyme Q₁₀ in the presence of a lysolecithin in an amount by weight not less than 0.7 times the amount thereof and an oil and fat, a method of increasing the absorbability of oxidized coenzyme Q₁₀ by ingesting oxidized coenzyme Q₁₀ in the presence of a lysolecithin, an oil and fat, and monoglyceride citrate, a method of increasing the absorbability of oxidized coenzyme Q₁₀ by ingesting oxidized coenzyme Q₁₀ in the presence of a lysolecithin, an oil and fat, and hexaglycerol monooleate, and a method of increasing the absorbability of oxidized coenzyme Q₁₀ by ingesting oxidized coenzyme Q₁₀ in the presence of a lysolecithin, an oil and fat, and tetraglycerol monolaurate.

### Examples

The present invention is hereinafter described in more detail by means of the following Examples, which, however, are not to be construed as limiting the present invention.

### Test Example 1

### <Preparation of test substance dosing solutions>

Test substance dosing solutions were prepared using the formulations shown in Table 1. In Examples 1 to 4 and Comparative Example 2, oxidized coenzyme Q₁₀ and each test substance were dissolved and mixed under warming at about 50 to 60°C, and these solutions were used as the dosing solutions. In Comparative Example 1, oxidized coenzyme Q₁₀ powder was added to a 0.5% (W/V) aqueous solution of carboxymethylcellulose sodium and dispersed by sonication to yield a dosing solution.

**[Table 1]**

| Dosing solution formulation (g/100.g dosing solution) | Ex. 1 | Ex. 2 | Ex. 3 | Ex. 4 | Comp. Ex. 1 | Comp. Ex. 2 |
|---|---|---|---|---|---|---|
| Oxidized coenzyme Q₁₀ | 2 | 2 | 2 | 2 | 2 | 2 |
| Lysolecithin | 10.8 | 10.8 | 10.8 | 10.8 | - | - |
| Lecithin | - | - | - | - | - | 10.8 |
| Hexaglycerol monooleate | - | 10.8 | - | - | - | |
| Tetraglycerol monolaurate | - | - | 10.8 | - | - | - |
| Decaglycerol monooleate | - | - | - | 10.8 | - | - |
| Safflower oil | 87.2 | 76.4 | 76.4 | 76.4 | - | 87.2 |
| 0.5% aqueous solution of carboxymethylcellulose | - | - | - | - | 98 | - |

Oxidized coenzyme Q₁₀: Manufactured by Kaneka Corporation
Lysolecithin: Degussa EMULTOP IP, HLB: 8
Lecithin: Degussa EMULPUR IP, HLB: 3 to 4
Hexaglycerol monooleate: Taiyo Kagaku Sunsoft Q-17F, HLB: 10.5
Tetraglycerol monolaurate: Sakamoto Yakuhin Kogyo SY-Glyster ML-310, HLB: 10
Decaglycerol monooleate: Sakamoto Yakuhin Kogyo SY-Glyster MO-3S,
HLB: 8.8
Safflower oil: Safflower oil containing high oleic acid, oleic acid content in constituent fatty acid : 76.6%

### <Experimental system>

The above-mentioned test substance dosing solution was orally administered to 13-week-old male Sprague-Dawley rats (supplier: Japan SLC, Inc.) at a dose of oxdized coenzyme Q₁₀ of 30 mg/kg. The blood was taken from each rat at 1, 2, 4, 8 and 24 hr after test substance administration. The obtained blood was centrifuged to plasma. Thereafter, an oxidization treatment from reduced coenzyme Q₁₀ to oxidized coenzyme Q₁₀ and an extraction treatment of oxidized coenzyme Q₁₀ were performed and plasma coenzyme Q₁₀ concentration was measured as oxidized coenzyme Q₁₀ using HPLC.

column; SYMMETRY C18 (manufactured by Waters) 250 mm (length) 4.6 mm (inner diameter)
mobile phase; C₂H₅OH:CH₃OH=4:3 (v:v)
detection wavelength; 210 nm
flow rate; 1 ml/min
retention time of oxidized coenzyme Q₁₀; 13.3 min.

### <Results>

The time course changes of plasma coenzyme Q₁₀ concentration after oral administration of the above-mentioned test substance dosing solution is shown in Table 2. In addition, the area under plasma coenzyme Q₁₀ concentration-time curve (AUC) from 1 hr to 24 hr after test substance administration are shown in Table 2.

**[Table 2]**

| | plasma coenzyme Q₁₀ concentration (µg/mL) | | | | | AUC 1-24 (µg/mL×hr) |
|---|---|---|---|---|---|---|
| | 1 hr | 2 hr | 4 hr | 8 hr | 24 hr | |
| Ex. 1 | 0.58 | 1.98 | 0.96 | 0.80 | 0.47 | 17.9 |
| Ex. 2 | 0.88 | 2.09 | 1.11 | 1.17 | 0.45 | 22.2 |
| Ex. 3 | 0.99 | 2.38 | 1.21 | 1.11 | 0.32 | 21.4 |
| Ex. 4 | 0.59 | 1.82 | 1.05 | 0.57 | 0.26 | 14.0 |
| Com. Ex. 1 | 0.54 | 0.59 | 0.89 | 0.61 | 0.27 | 12.0 |
| Com. Ex. 2 | 0.95 | 1.97 | 0.74 | 0.66 | 0.44 | 15.8 |

| | | | | | | |
|---|---|---|---|---|---|---|
| The data shows an average value of each n=5. AUC 1-24: the area under plasma coenzyme Q₁₀ concentration-time curve (AUC) from 1 to 24 hours after administration of test substance | | | | | | |

Compared to Comparative Example 1, wherein oxidized coenzyme Q₁₀ was dispersed in an aqueous solution of carboxymethylcellulose sodium, Example 1, which comprises oxidized coenzyme Q₁₀ and lysolecithin, showed a plasma coenzyme Q₁₀ maximum plasma concentration 2.22 times and an AUC value 1.49 times; it was confirmed that the composition for oral administration of the present invention (Example 1), which comprises oxidized coenzyme Q₁₀ and lysolecithin, exhibits extremely high absorbability. It was also shown that the composition of Comparative Example 2, which comprises oxidized coenzyme Q₁₀ and lecithin, also exhibited higher absorbability than Comparative Example 1, but was less effective than the composition of the present invention, which comprises lysolecithin (Example 1).
It was also confirmed that the compositions comprising hexaglycerol monooleate or tetraglycerol monolaurate as the surfactant (Examples 2 and 3) exhibit even higher oral absorbability.

### Test Example 2

### <Preparation of test substance dosing solutions>

Using the formulations shown in Table 3, soybean-derived lysolecithin (Degussa EMULTOP IP) and safflower oil (safflower oil containing high oleic acid, oleic acid content in constituent fatty acid : 76.6%) were dissolved under warming at about 50 to 60°C, and blended by sonication. Next, oxidized coenzyme Q₁₀ (Kaneka Corporation) was added to the lysolecithin/safflower oil mixture, and dissolved under warming at about 50 to 60°C, and they were blended by sonication, to yield dosing solutions having oxidized coenzyme Q₁₀ concentrations of 2 wt% (the weight ratio of oxidized coenzyme Q₁₀/lysolecithin was 1/0.7 to 14.3), respectively (Examples 5 to 10). For control, oxidized coenzyme Q₁₀ was added only to safflower oil (safflower oil containing high oleic acid, oleic acid content in constituent fatty acid : 76.6%) without using lysolecithin, and dissolved under warming at about 50 to 60°C, and they were blended by sonication, to yield a dosing solution having an oxidized coenzyme Q₁₀ concentration of 2 wt%

### (Comparative Example 3).

### <Experiment system>

The above-mentioned test substance dosing solution was orally administered to 11-week-old male Sprague-Dawley rats (supplier: Japan SLC, Inc.) at a dose of oxidized coenzyme Q₁₀ of 30 mg/kg. The blood was taken from each rat at 1, 2, 4, 8 and 24 hr after test substance administration. The obtained blood was centrifuged to plasma. Thereafter, plasma coenzyme Q₁₀ concentration was measured using HPLC under the same conditions as in Test Example 1. The plasma coenzyme Q₁₀ concentration before administration was data processed as 0 µg/ml.

### <Results>

Maximum plasma coenzyme Q₁₀ concentration values after oral administration of the above-described test substance dosing solutions and area under the plasma coenzyme Q₁₀ concentration-time curve (AUC) values from 0 to 24 hours after administration of test substance are shown in Table 3.

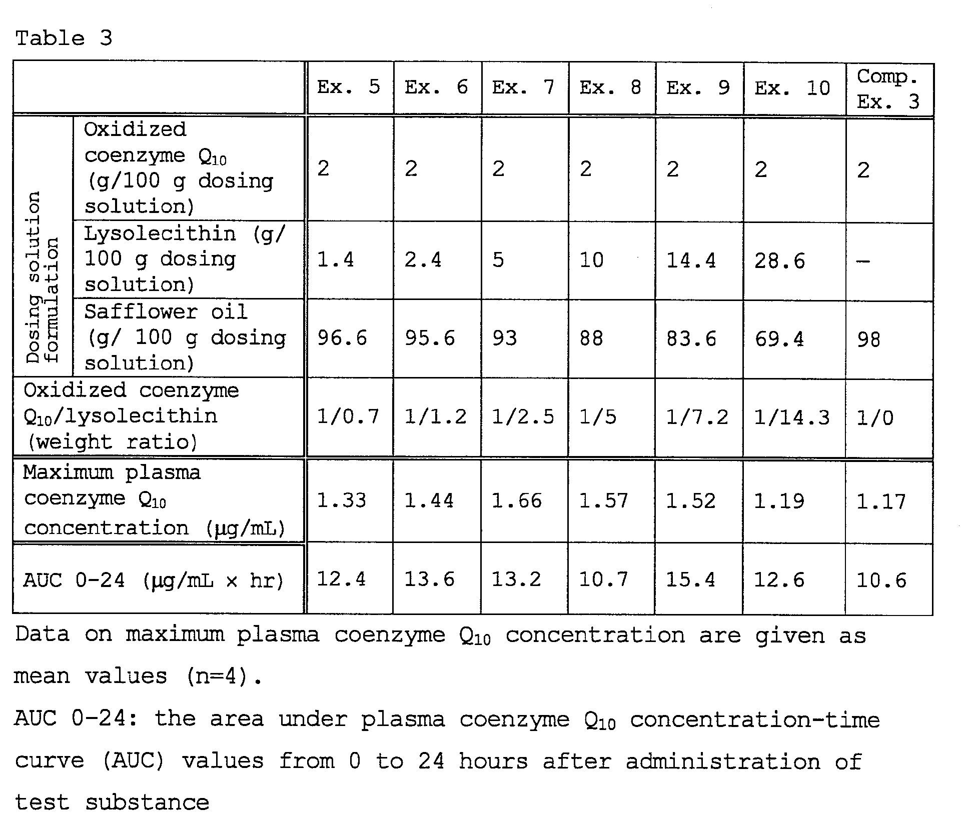

A comprehensive evaluation of the results of both maximum plasma coenzyme Q₁₀ concentration and area under the plasma coenzyme Q₁₀ concentration-time curve showed that the compositions of Examples 5 to 10, which consist of oxidized coenzyme Q₁₀, lysolecithin and an oil and fat, have higher absorbability of oxidized coenzyme Q₁₀ than the composition of Comparative Example 3, which consists of oxidized coenzyme Q₁₀ and an oil and fat only. An evaluation based on maximum plasma coenzyme Q₁₀ concentration data alone showed that oxidized coenzyme Q₁₀ exhibits particularly high absorbability when prepared as a composition wherein the weight ratio of oxidized coenzyme Q₁₀ and lysolecithin is between 1/1.2 and 1/7.2.

### Test Example 3

### <Preparation of test substance dosing solutions>

The various oils and fats shown in Table 4 and soybean-derived lysolecithin (Degussa EMULTOP IP) were dissolved under warming at about 50 to 60°C to obtain a weight ratio of lysolecithin/oil and fat of 1/8, and blended by sonication. Next, oxidized coenzyme Q₁₀ (Kaneka Corporation) was added to the various oils and fats/lysolecithin thus obtained, and dissolved under warming at about 50 to 60°C, and they were blended by sonication, to yield dosing solutions having an oxidized coenzyme Q₁₀ concentration of 2 wt% (the weight ratio of oxidized coenzyme Q₁₀/lysolecithin/ oil and fat was about 2/10.8/87.2), respectively.

**[Table 4]**

| Dosing solution formulation (g/100 g dosing solution) | Ex. 11 | Ex. 12 | Ex. 13 | Ex. 14 | Ex. 15 | Ex. 16 | Ex. 17 | Ex. 18 |
|---|---|---|---|---|---|---|---|---|
| Oxidized coenzyme Q₁₀ | 2 | 2 | 2 | 2 | 2 | 2 | 2 | 2 |
| Lysolecithin | 10.8 | 10.8 | 10.8 | 10.8 | 10.8 | 10.8 | 10.8 | 10.8 |
| Dietary safflower oil (oleic acid content in constituent fatty acids not more than 30%) | 87.2 | - | - | - | - | - | - | - |
| Safflower oil containg high oleic acid content (oleic acid content in constituent fatty acid : 76.6%) | - | 87.2 | - | - | - | - | - | - |
| Olive oil | - | - | 87.2 | - | - | - | - | - |
| Almond oil | - | - | - | 87.2 | - | - | - | - |
| Sesame oil | - | - | - | - | 87.2 | - | - | - |
| Rice oil | - | - | - | - | - | 87.2 | - | - |
| Cottonseed oil | - | - | - | - | - | - | 87.2 | - |
| Soybean oil | - | - | - | - | - | - | - | 87.2 |

### <Experiment system>

The above-mentioned test substance dosing solution was orally administered to 11-week-old male Sprague-Dawley rats (supplier: Japan SLC, Inc.) at a dose of oxidized coenzyme Q₁₀ of 30 mg/kg. The blood was taken from each rat at 1, 2, 4, 8 and 24 hr after test substance administration. The obtained blood was centrifuged to plasma. Thereafter, plasma coenzyme Q₁₀ concentration was measured using HPLC under the same conditions as in Test Example 1. The plasma coenzyme Q₁₀ concentration before administration was data processed as 0 µg/ml.

### <Results>

Maximum plasma coenzyme Q₁₀ concentration values after oral administration of the above-described test substance dosing solutions and area under the plasma coenzyme Q₁₀ concentration-time curve (AUC) values from 0 to 24 hours after administration of test substance are shown in Table 5.

**Table 5**

| | Ex. 11 | Ex. 12 | Ex. 13 | Ex. 14 | Ex. 15 | Ex. 16 | Ex. 17 | Ex. 18 |
|---|---|---|---|---|---|---|---|---|
| Choice of oil and fat | Dietary safflower oil | Safflower oil containing high oleic acid | Olive oil | Almond oil | Sesame oil | Rice oil | Cottonseed oil | Soybean oil |
| Maximum plasma coenzyme Q₁₀ concentration (µg/mL) | 1.17 | 2.53 | 1.66 | 2.38 | 1.44 | 1.42 | 2.38 | 1.23 |
| AUC 0-24 (µg/mL × hr) | 13.9 | 15.1 | 12.0 | 14.5 | 11.0 | 9.4 | 17.0 | 11.4 |

| | | | | | | | | |
|---|---|---|---|---|---|---|---|---|
| Data are given as mean values (n=4). AUC 0-24: the area under plasma coenzyme Q₁₀ concentration-time curve (AUC) values from 0 to 24 hours after administration of test substance | | | | | | | | |

It was shown that maximum plasma coenzyme Q₁₀ concentration and AUC values increased remarkably in a composition using safflower oil containing high oleic acid, almond oil, and cottonseed oil, of the compositions of Examples 11 to 18, which consist of oxidized coenzyme Q₁₀, a lysolecithin and various oils and fats.
Hence, it was shown that by preparing a composition consisting of oxidized coenzyme Q₁₀, a lysolecithin and various oils and fats, and containing a particular oil and fat, the absorbability is increased dramatically.

### Test Example 4

### <Preparation of test substance dosing solutions>

Test substance dosing solutions were prepared using the formulations shown in Table 6. In Examples 19 to 23 and Comparative Example 6, ingredients other than oxidized coenzyme Q₁₀ were first dissolved under warming at about 50 to 60°C, and blended by sonication, using the ratios shown in Table 6, after which oxidized coenzyme Q₁₀ was added, and dissolved under warming at about 50 to 60°C, and they were blended by sonication, to yield dosing solutions having an oxidized coenzyme Q₁₀ concentration of 2 wt%, respectively. In Comparative Examples 4 and 5, oxidized coenzyme Q₁₀ was added to each of safflower oil and monoglyceride citrate, and dissolved under warming at about 50 to 60°C, and they were blended by sonication, to yield dosing solutions having an oxidized coenzyme Q₁₀ concentration of 2 wt%, respectively. The lysolecithin used was soybean-derived lysolecithin (Degussa EMULTOP IP); the oil and fat used was safflower oil (safflower oil containing high oleic acid, oleic acid content in constituent fatty acid : 76.6%).

### <Experiment system>

10-week-old male Sprague-Dawley rats (supplier: Japan SLC, Inc.) were fasted from the evening of one day before the experiment and the above-mentioned test substance dosing solution was orally administered at a dose of oxidized coenzyme Q₁₀ of 30 mg/kg. The blood was taken from each rat at 1, 2, 4, 8 and 24 hr after test substance administration. The obtained blood was centrifuged to plasma. Thereafter, plasma coenzyme Q₁₀ concentration was measured using HPLC under the same conditions as in Test Example 1. The plasma coenzyme Q₁₀ concentration before administration was data processed as 0 µg/ml in this test. For reference, the same test substance dosing solutions as those in Example 20 were administered to non-fasted rats, and measurements and data processing were performed in the same manner (Example 23).

### <Results>

Maximum plasma coenzyme Q₁₀ concentration values after oral administration of the above-described test substance dosing solutions and area under the plasma coenzyme Q₁₀ concentration-time curve (AUC) values from 0 to 24 hours after administration of test substance are shown in Table 6.

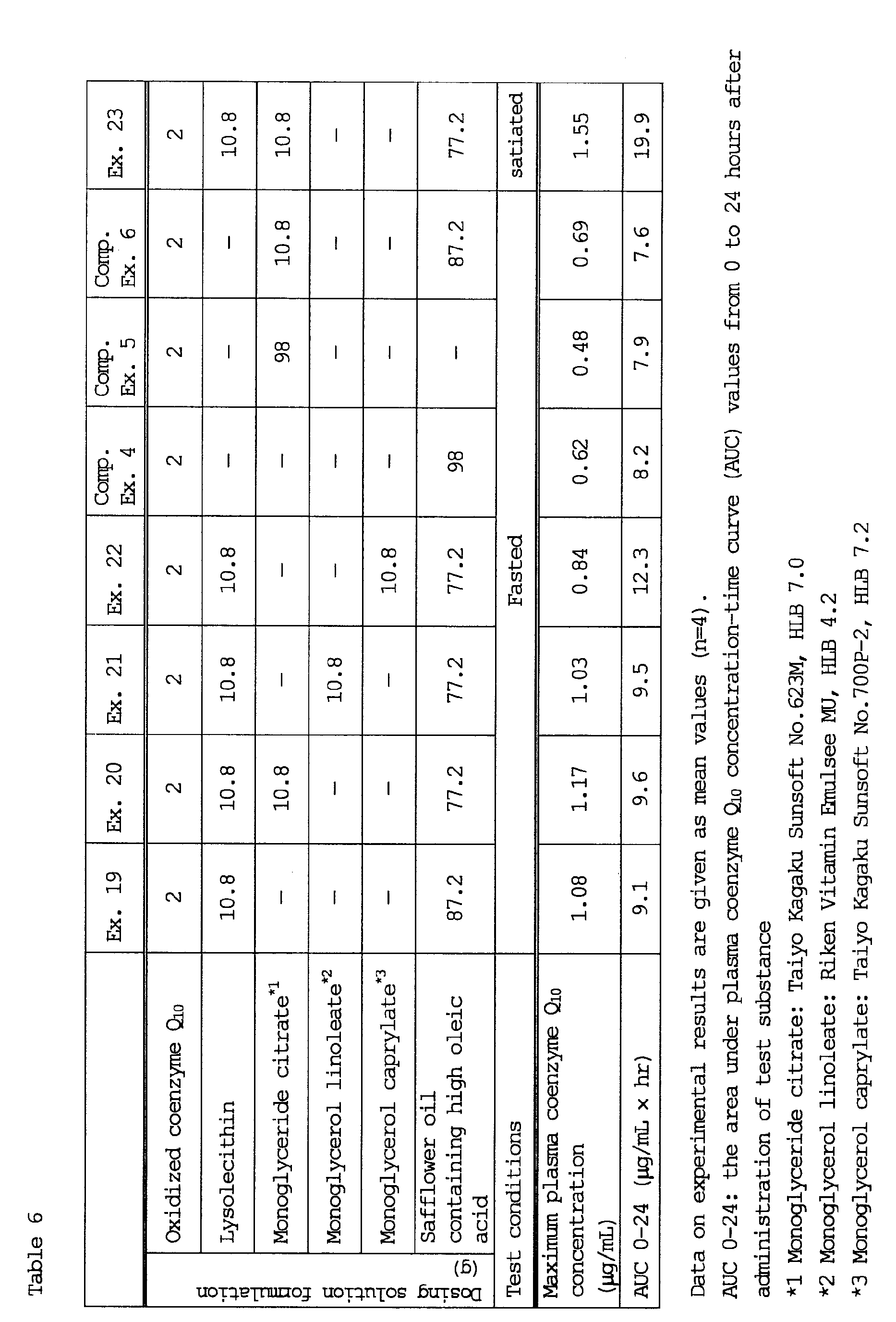

Generally, liposoluble substances such as coenzyme Q₁₀ are absorbed more by ingestion with food but hardly absorbed during fasting. From the above-mentioned results, it has been clarified that the composition of the present invention containing oxidized coenzyme Q₁₀, lysolecithin and oil and fat (Example 19 to 22) shows superior absorbability during fasting, namely, in hungry rats.

### Test Example 5

### <Preparation of test substance dosing solutions>

Test substance dosing solutions were prepared using the formulations shown in Table 7. In Example 24, oxidized coenzyme Q₁₀ powder and soybean-derived lysolecithin (Degussa EMULTOP IP) were blended to obtain an oxidized coenzyme Q₁₀/lysolecithin weight ratio of 1/0.7, and safflower oil (safflower oil containing high oleic acid, oleic acid content in constituent fatty acid: 76.6%) was further added, and dissolved under warming at about 50 to 60°C, and they were blended by sonication, to yield dosing solutions. In Comparative Examples 7 and 8, oxidized coenzyme Q₁₀ powder alone, or oxidized coenzyme Q₁₀ powder and soybean-derived lysolecithin (Degussa EMULTOP IP) were added to a 0.5% (W/V) aqueous solution of carboxymethylcellulose sodium and dispersed by sonication to yield dosing solutions. All these dosing solutions were prepared to obtain an oxidized coenzyme Q₁₀ concentration of 2 wt%.

### <Experimental system>

Each of the above-described test substance dosing solutions was orally administered at a dose of 30 mg/kg oxidized coenzyme Q₁₀ to 11-week-old male Sprague-Dawley rats (supplier: Japan SLC, Inc.). At 1, 2, 4, 8, and 24 hours after administration of test substance, blood was taken from each rat. The obtained blood was centrifuged to plasma. Subsequently, HPLC was performed under the same conditions as Test Example 1 to determine the coenzyme Q concentration in the plasma. In this experiment, the plasma coenzyme Q₁₀ concentration before administration was data processed as 0 µg/mL.

### <Results>

Maximum plasma coenzyme Q₁₀ concentrations after oral administration of the above-described test substance dosing solutions and area under the plasma coenzyme plasma coenzyme Q₁₀ concentration-time curve (AUC) values from 0 to 24 hours after administration of test substance are shown in Table 7.

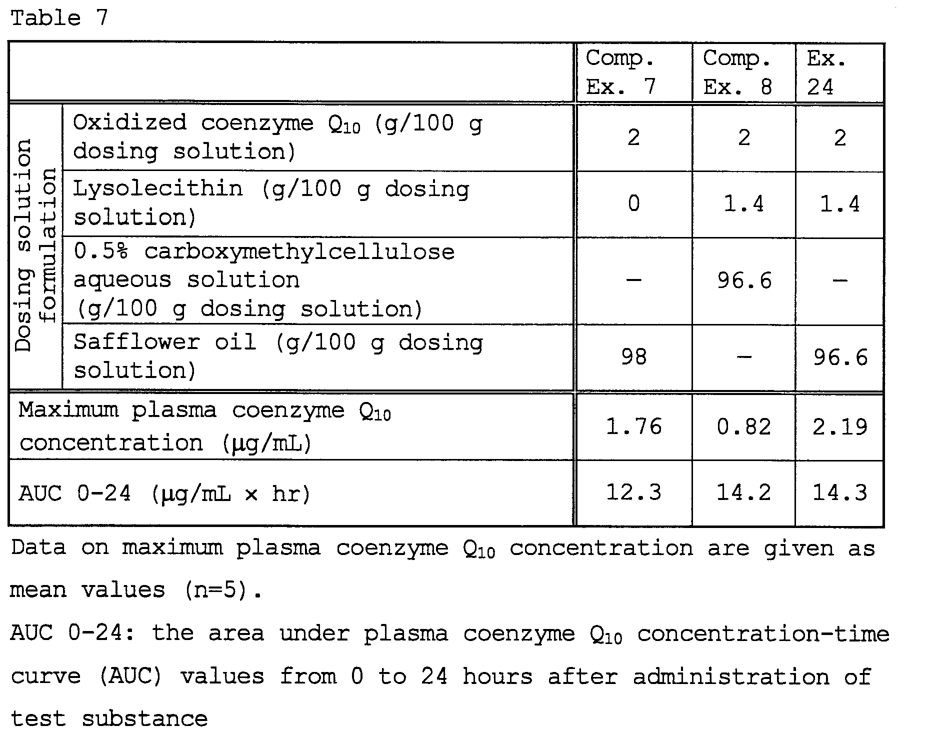

The results shown above demonstrate that not only a lysolecithin, but also an oil and fat, is necessary to improve the absorbability of oxidized coenzyme Q₁₀.

### Preparation Example 1: Soft capsule 1

Oxidized coenzyme Q₁₀ (Kaneka Corporation) was added to a mixture consisting of safflower oil (safflower containing high oleic acid, oleic acid content in constituent fatty acid : 76.6%), a lysolecithin (Degussa EMULTOP IP), and yellow beeswax at 60°C. The mixture obtained was treated by a conventional method to yield a gelatin soft capsule preparation consisting of the ingredients shown below.

| | |
|---|---|
| oxidized coenzyme Q₁₀ | 10 parts by weight |
| lysolecithin | 10 parts by weight |
| beeswax | 5 parts by weight |
| safflower oil | 75 parts by weight |

### Preparation Example 2: Soft capsule 2

Oxidized coenzyme Q₁₀ (Kaneka Corporation) was added to a mixture consisting of a middle-chain fatty acid triglycerides, a lysolecithin (Degussa EMULTOP IP) and yellow beeswax was added at 40°C. The mixture obtained was treated by a conventional method to yield a gelatin soft capsule preparation consisting of the ingredients shown below.

| | |
|---|---|
| oxidized coenzyme Q₁₀ | 10 parts by weight |
| lysolecithin | 10 parts by weight |
| beeswax | 5 parts by weight |
| medium chain triglyceride | 75 parts by weight |

### Preparation Example 3: Soft capsule 3

Oxidized coenzyme Q₁₀ (Kaneka Corporation) was added to a mixture consisting of a middle-chain fatty acid triglyceride, a lysolecithin (Degussa EMULTOP IP), a lecithin (Degussa EMULPUR IP), and yellow beeswax at 40°C. The mixture obtained was treated by a conventional method to yield a gelatin soft capsule preparation consisting of the ingredients shown below.

| | |
|---|---|
| oxidized coenzyme Q₁₀ | 2 parts by weight |
| lysolecithin | 1.4 parts by weight |

| | |
|---|---|
| lecithin | 10.8 parts by weight |
| beeswax | 5 parts by weight |
| medium chain triglyceride | 75 parts by weight |

### Preparation Example 4: Soft capsule 4

Oxidized coenzyme Q₁₀ (Kaneka Corporation) was added to a mixture consisting of safflower oil (safflower oil containing high oleic acid, oleic acid content in constituent fatty acid : 76.6%), a lysolecithin (Degussa EMULTOP IP), hexaglycerol monooleate (Taiyo Kagaku Sunsoft Q-17F), and yellow beeswax at 50°C. The mixture obtained was treated by a conventional method to yield a gelatin soft capsule preparation consisting of the ingredients shown below.

| | |
|---|---|
| oxidized coenzyme Q₁₀ | 2 parts by weight |
| lysolecithin | 10.8 parts by weight |
| hexaglycerol monooleate | 10.8 parts by weight |
| beeswax | 5 parts by weight |
| safflower oil | 75 parts by weight |

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2006-275501 filed in Japan and US provisional application No. 60/828490, the contents of which are incorporated in full herein by this reference.

## Claims

1. A composition for oral administration comprising an oxidized coenzyme Q₁₀ represented by the structural formula (1): 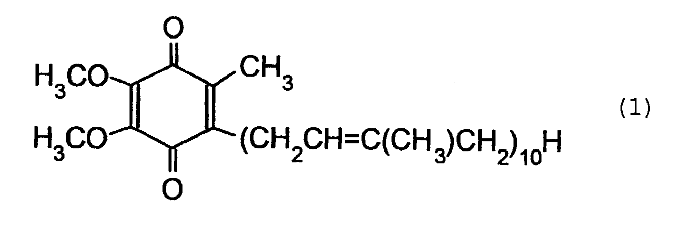 a lysolecithin and an oil and fat, wherein the weight ratio of the lysolecithin to the oxidized coenzyme Q₁₀ is not less than 0.7.

2. The composition for oral administration of claim 1, wherein the weight ratio of the lysolecithin to the oxidized coenzyme Q₁₀ is not less than 1.2.

3. The composition for oral administration of claim 1 or 2, further comprising a lecithin.

4. The composition for oral administration of claim 3, wherein the weight ratio of the total of the lysolecithin and the lecithin to the oxidized coenzyme Q₁₀ is not less than 1.2.

5. The composition for oral administration of any one of claims 1 to 4, wherein the lysolecithin is a lysolecithin derived from at least one kind of lecithin selected from the group consisting of soybean lecithin, egg-yolk lecithin, corn lecithin, cottonseed oil lecithin, rapeseed lecithin, phosphatidylcholine, phosphatidylethanolamine and phosphatidylglycerol.

6. The composition for oral administration of any one of claims 3 to 5, wherein the lecithin is at least one kind of lecithin selected from the group consisting of soybean lecithin, egg-yolk lecithin, corn lecithin, cottonseed oil lecithin, rapeseed lecithin, phosphatidylcholine, phosphatidylethanolamine and phosphatidylglycerol.

7. The composition for oral administration of any one of claims 1 to 6, wherein the oil and fat is at least one kind of oil and fat selected from the group consisting of coconut oil, palm oil, palm kernel oil, linseed oil, camelia oil, unmilled rice germ oil, avocado oil, rapeseed oil, rice oil, peanut oil, corn oil, wheat germ oil, soybean oil, perilla oil, cottonseed oil, sunflower oil, kapok oil, evening primrose oil, shea butter, sal butter, cacao butter, sesame oil, safflower oil, olive oil, almond oil, lard, milk fat, fish oil, beef tallow, processed oils and fats therefrom, middle-chain fatty acid triglycerides, and hydrolysates thereof.

8. The composition for oral administration of claim 7, wherein the oil and fat is at least one kind of oil and fat selected from the group consisting of safflower oil, almond oil and cottonseed oil.

9. The composition for oral administration of any one of claims 1 to 6, wherein the oil and fat is an oil and fat wherein oleic acid accounts for not less than 30 wt% of the constituent fatty acids thereof.

10. The composition for oral administration of any one of claims 1 to 9, further comprising a surfactant.

11. The composition for oral administration of claim 10, wherein the surfactant is at least one kind of surfactant selected from the group consisting of glycerol fatty acid esters, sucrose fatty acid esters, organic acid monoglycerides, sorbitan fatty acid esters, polyoxyethylene sorbitan fatty acid esters, propylene glycol fatty acid esters, condensed ricinoleinic acid polyglycerides, and saponin.

12. The composition for oral administration of claim 11, wherein the surfactant is at least one kind of glycerol fatty acid ester and organic acid monoglyceride.

13. The composition for oral administration of claim 12, wherein the glycerol fatty acid ester is hexaglycerol monooleate and/or tetraglycerol monolaurate.

14. The composition for oral administration of claim 12, wherein the organic acid monoglyceride is monoglyceride citrate.

15. The composition for oral administration of any one of claims 1 to 14, wherein the content of oxidized coenzyme Q₁₀ in the composition is not less than 0.01 wt% to total weight of the composition.

16. The composition for oral administration of any one of claims 1 to 15, which is in the form of a liquid or a slurry.

17. The composition for oral administration of any one of claims 1 to 15, which is in the form of a solid.

18. The composition for oral administration of any one of claims 1 to 17, which is a pharmaceutical or a quasi-drug.

19. The composition for oral administration of any one of claims 1 to 17, which is a food.

20. The composition for oral administration of any one of claims 1 to 17, which is a feed, a prey or a pet food.

21. The composition for oral administration of any one of claims 17 to 20, which is in form of tablets, powders, chewable tablets, pills, or capsules.

22. The composition for oral administration of claim 21, wherein the capsules are soft capsule preparation.

23. A composition for oral administration comprising oxidized coenzyme Q₁₀, a lysolecithin, an oil and fat, and monoglyceride citrate.

24. A composition for oral administration comprising oxidized coenzyme Q₁₀, a lysolecithin, an oil and fat, and hexaglycerol monooleate.

25. A composition for oral administration comprising oxidized coenzyme Q₁₀, a lysolecithin, an oil and fat, and tetraglycerol monolaurate.

26. The composition for oral administration of any one of claims 23 to 25, wherein the weight ration of the lysolecithin to the oxidized coenzyme Q₁₀ is not less than 0.25.

27. The composition for oral administration of any one of claims 23 to 26, wherein the oil and fat is at least one kind of oil and fat selected from the group consisting of safflower oil, almond oil, and cottonseed oil.

28. The composition for oral administration of any one of claims 23 to 26, wherein the oil and fat is an oil and fat wherein oleic acid amounts for not less than 30 wt% of the constituent fatty acids thereof.

29. The composition for oral administration of any one of claims 23 to 28, which is in the form of a soft capsule preparation.

30. A method of increasing the absorbability of oxidized coenzyme Q₁₀, comprising preparing the oxidized coenzyme Q₁₀ as a composition ingestible in the presence of a lysolecithin in an amount by weight not less than 0.7 times the amount thereof and an oil and fat.

31. A method of increasing the absorbability of oxidized coenzyme Q₁₀, comprising preparing the oxidized coenzyme Q₁₀ as a composition ingestible in the presence of a lysolecithin, an oil and fat, and monoglyceride citrate.

32. A method of increasing the absorbability of oxidized coenzyme Q₁₀, comprising preparing the oxidized coenzyme Q₁₀ as a composition ingestible in the presence of a lysolecithin, an oil and fat, and hexaglycerol monooleate.

33. A method of increasing the absorbability of oxidized coenzyme Q₁₀, comprising preparing the oxidized coenzyme Q₁₀ as a composition ingestible in the presence of a lysolecithin, an oil and fat, and tetraglycerol monolaurate.
